# EUROPEAN PATENT SPECIFICATION

(11) **EP 1 720 497 B1**
(45) Date of publication and mention of the grant of the patent: **10.09.2008**
(21) Application number: 05702042.2
(22) Date of filing: 27.01.2005
(51) Int. Cl.: A61F 5/44

(54) **Personal urinal**
Persönliches Urinal
Dispositif urinaire personnel

(30) Priority: 01.03.2004 GB 0404603
(43) Date of publication of application: 15.11.2006
(73) Proprietor: Campbell, James Albert Alexander, East Dulwich London SE22 9PD (GB); Campbell, Joyce Veronica, East Dulwich London SE22 9PD (GB); Campbell, Rushworth Oliver Charles, East Dulwich London SE22 9DP (GB)
(72) Inventor: Campbell, James Albert Alexander, East Dulwich London SE22 9PD (GB); Campbell, Joyce Veronica, East Dulwich London SE22 9PD (GB); Campbell, Rushworth Oliver Charles, East Dulwich London SE22 9DP (GB)
(74) Representative: Paget, Hugh Charles Edward
(86) International application number: PCT/GB2005/000290
(87) International publication number: WO 2005/084594

(56) References cited:
- EP-A- 0 287 441
- EP-A- 0 610 638
- WO-A-00/33773
- GB-A- 2 239 675
- GB-A- 2 384 711
- US-A- 5 342 583

## Description

The invention relates to incontinence devices.

GB 2,384,711 describes a urinal device having a groin-shaped collection bag. The device further comprises a vented appendage which is connected to the groin bag by a flexible valved inlet. The vented appendage has a motor for drawing urine from the appendage to the groin bag. The motor is connected to a battery held in a compartment of the groin bag.

The present invention provides an incontinence device as set out in claim 1.

The invention will now be described in more detail with reference to the accompanying drawings.

Figs. 19 and 29-50 show embodiments of the incontinence device of the invention.

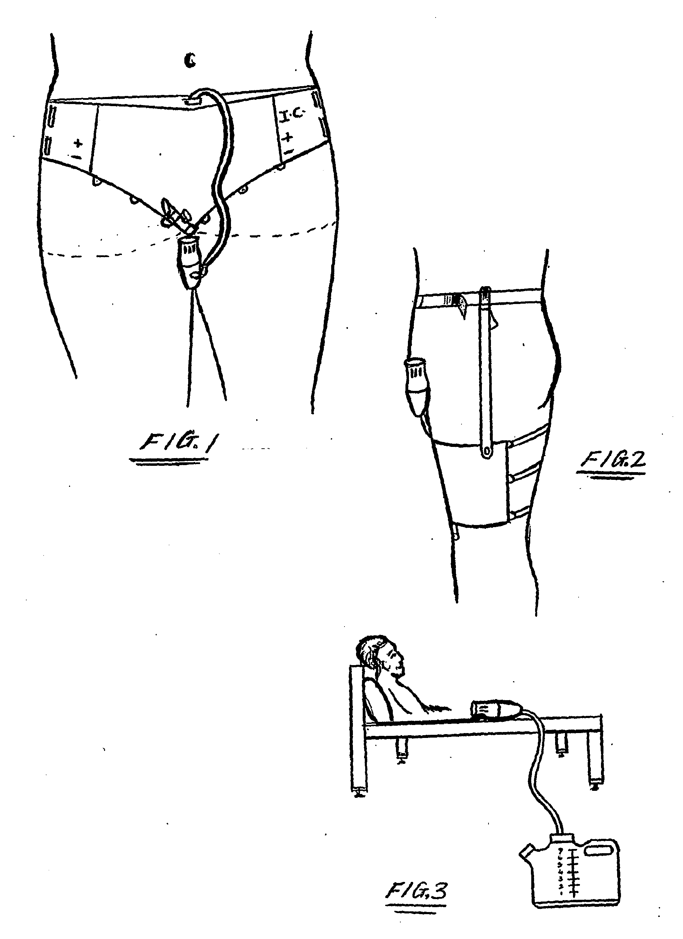

Figs. 1-18 and 20-28 show incontinence devices useful for understanding the incontinence device of the present invention.

PAGE 1/11, shows three applications of the male configuration of the incontinence device, wherein Fig. 1 is a motorised application of the system. Fig. 2 a leg bag application of the system and Fig. 3 a free standing application of the system with calibrated container.

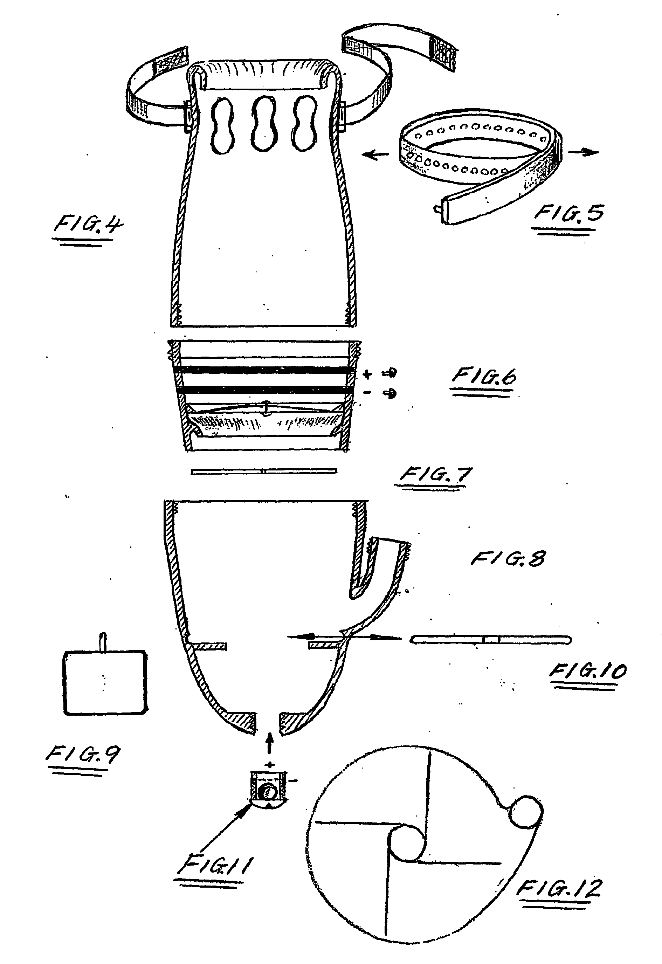

PAGE2/11, shows a cross section through the dismantled male system wherein Fig. 4 is the vented appendage with internal rolled upper edge and low tension elasticated adjuster. Fig. 5 shows the expandable ring, Fig. 6 shows the lockable liquid detector with the non-return valve and circumferential anti-rise rim. Fig. 7 shows the removed flexible gland of the non-return valve. Fig. 8 shows the empty housing, Fig. 9 shows the motor, Fig. 10 shows a press fit base plate, Fig. 11 is the removable levelling device and Fig. 12 is a plan revealing the fin/propeller position.

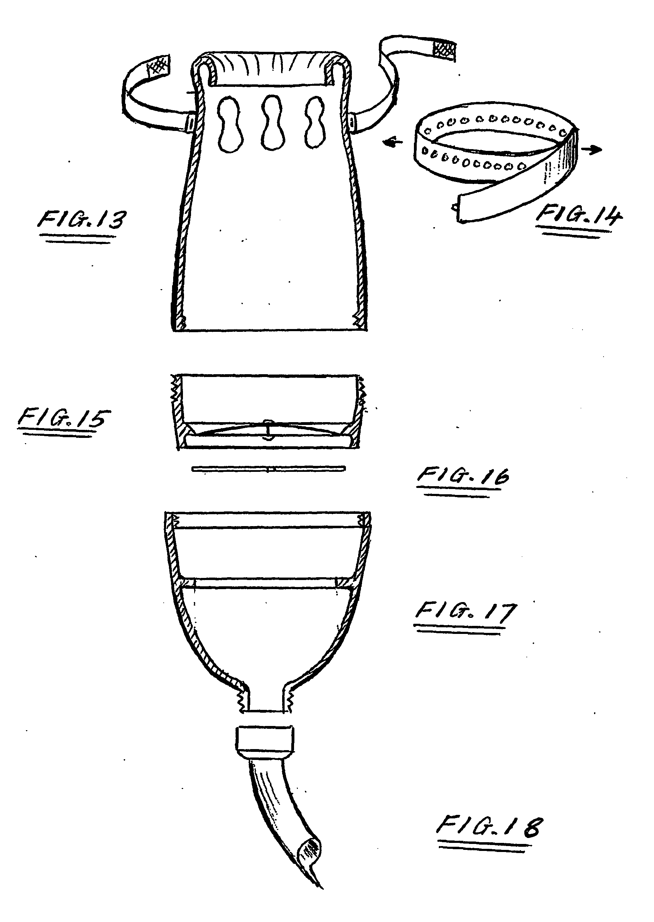

PAGE 3/11, shows a cross section through the male gravity fed system, wherein Fig. 13 is the vented appendage with internal rolled upper edge and low tension elasticated adjuster, Fig. 14 shows the expandable ring, Fig. 15 is a self-locking non-return valve, Fig. 16 shows the removed flexible gland of the non-return valve, Fig. 17 shows the empty housing and Fig. 18 is a detachable outlet that will lead to a leg bag or free standing container.

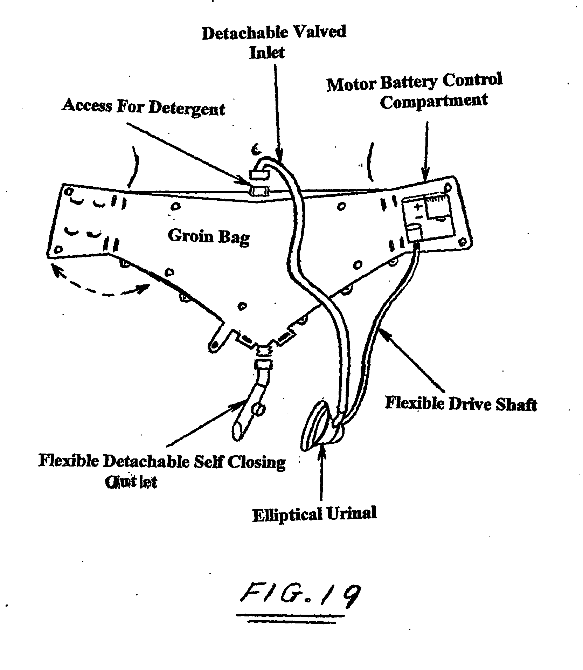

PAGE4/11, Fig. 19 shows a labelled female configuration of the incontinence system.

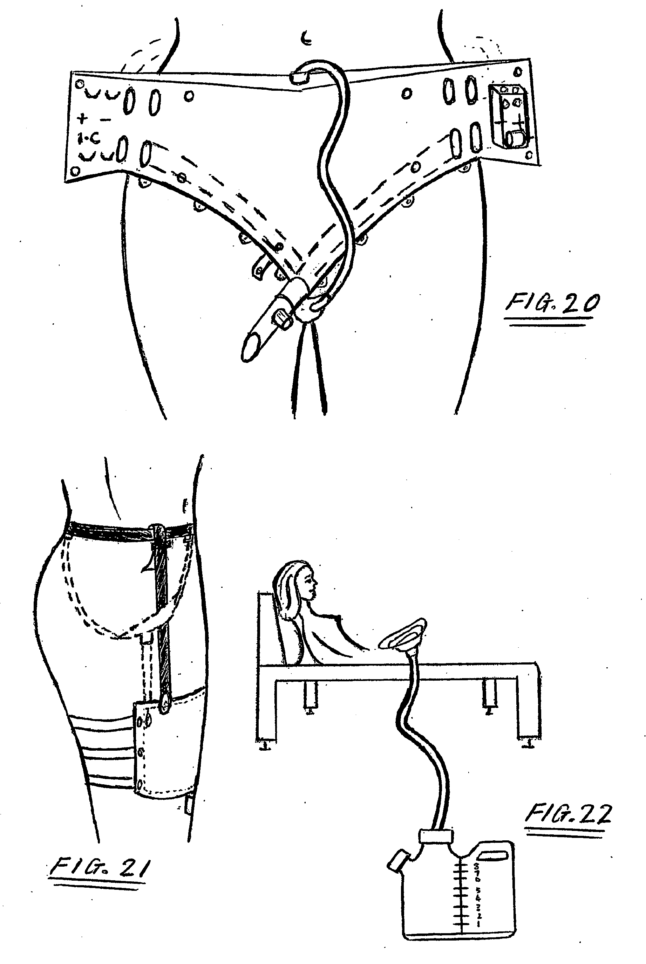

PAGE5/11, shows three applications of female configuration of the incontinence device wherein Fig. 20 is the motorised application of the system. Fig. 21 the gravity fed application of the system incorporating a leg bag. Fig. 22 the gravity fed application of the system with free standing container.

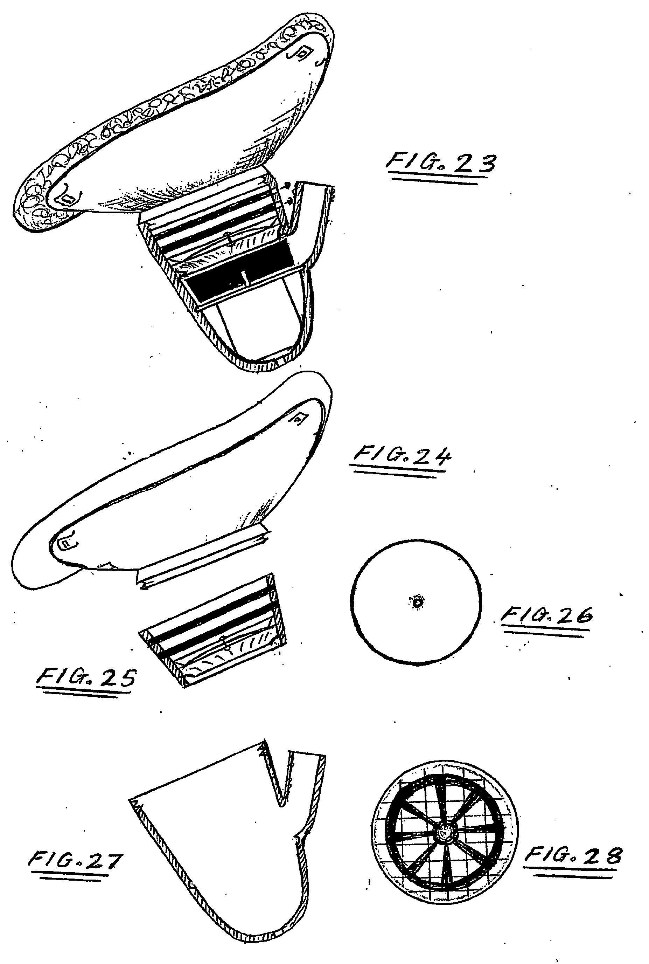

PAGE 6/11, Fig. 23 shows a cross section through the assembled direct drive system.

Fig. 24 shows the removed elliptical bowl with the pervious membrane attached, Fig. 25 shows the lockable liquid detector with the non-return valve and circumferential anti- rise rim, Fig. 26 is a press fit base plate, Fig. 27 shows the empty housing and Fig. 28 is a plan of the non-return valve and liquid detector.

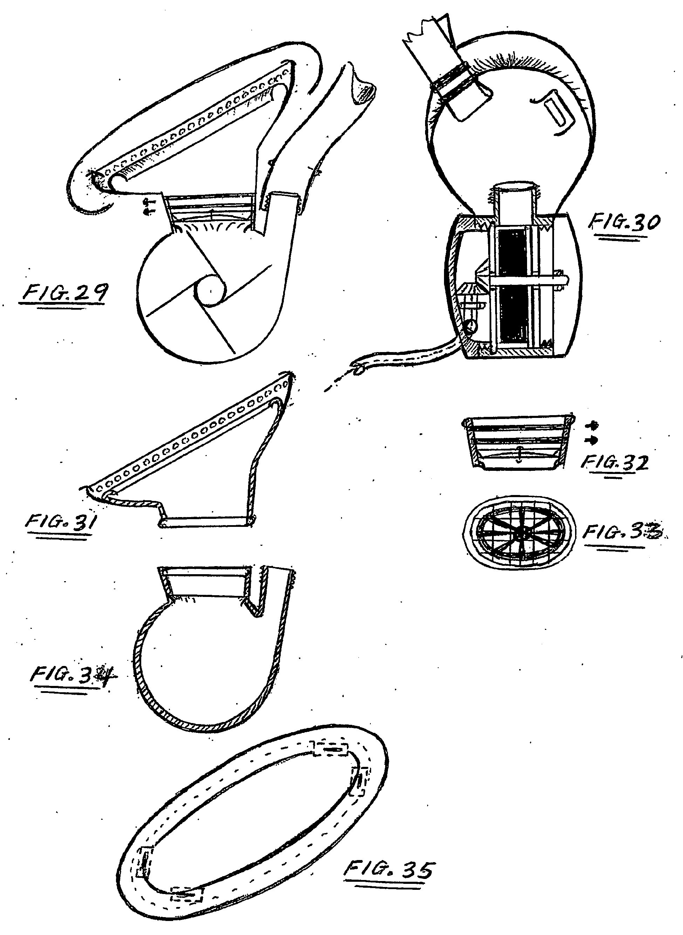

PAGE 7/11, Fig. 29 shows a cross section through the flexible drive system revealing the position of the impeller and the flexible, detachable outlet that will lead to the groin bag. Fig. 30 shows the system with the flexible drive shaft with 1 of 4 adjustable anchoring straps. Fig. 31 shows the cross section through the removed elliptical bowl with its elevated circumferential perforated edge. Fig. 32 and Fig. 33 show a section and plan of the non-return valve and the liquid detector with terminal screws. Fig. 34 shows the empty housing and Fig. 35 shows the removed pervious membrane.

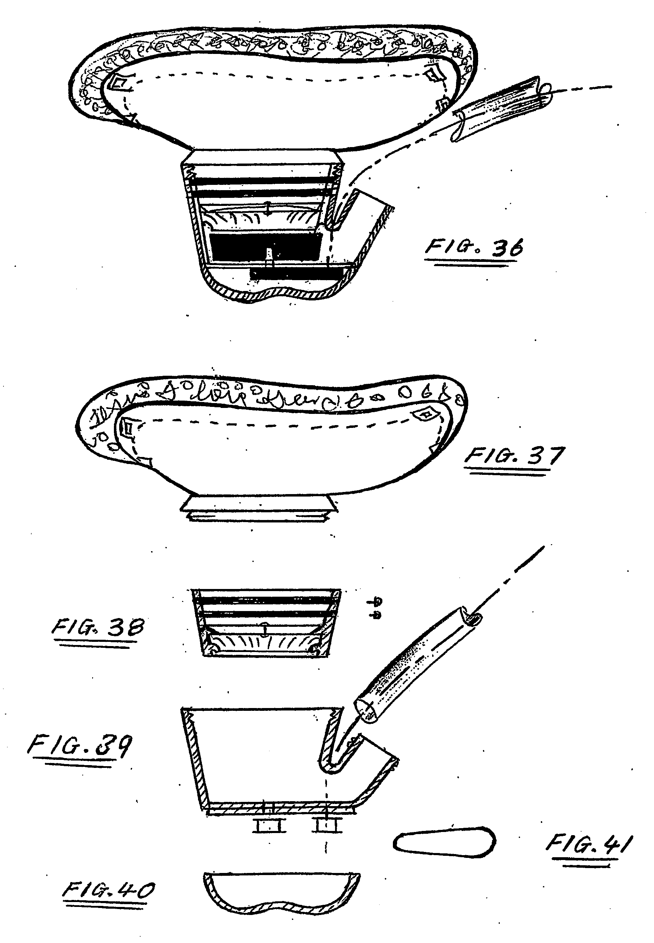

PAGE 8/11, Fig. 36 shows a cross section through the assembled belt driven device. Fig.37 shows the removed elliptical bowl with the pervious membrane. Fig.38 shows the liquid detector and the non-return valve. Fig. 39 shows the empty housing, Fig. 40 shows the female profile base plate and Fig. 41 shows the removed belt.

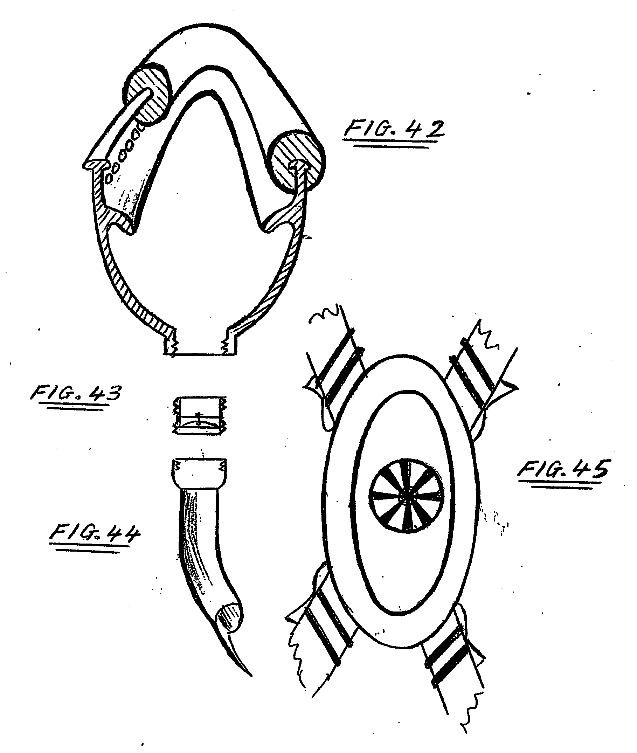

PAGE9/11, Fig. 42 shows a cross section through the gravity fed system, revealing the profile of the elliptical bowl with flexible detachable membrane, its elevated perforated edge and the slope of the internal circumferential rim. Fig. 43 is a non-return valve and Fig. 44 is a detachable inlet that will lead to a leg bag or a free standing container. Fig. 45 is a plan of the system revealing the 4 adjustable anchoring straps, the pervious membrane and the non return valve.

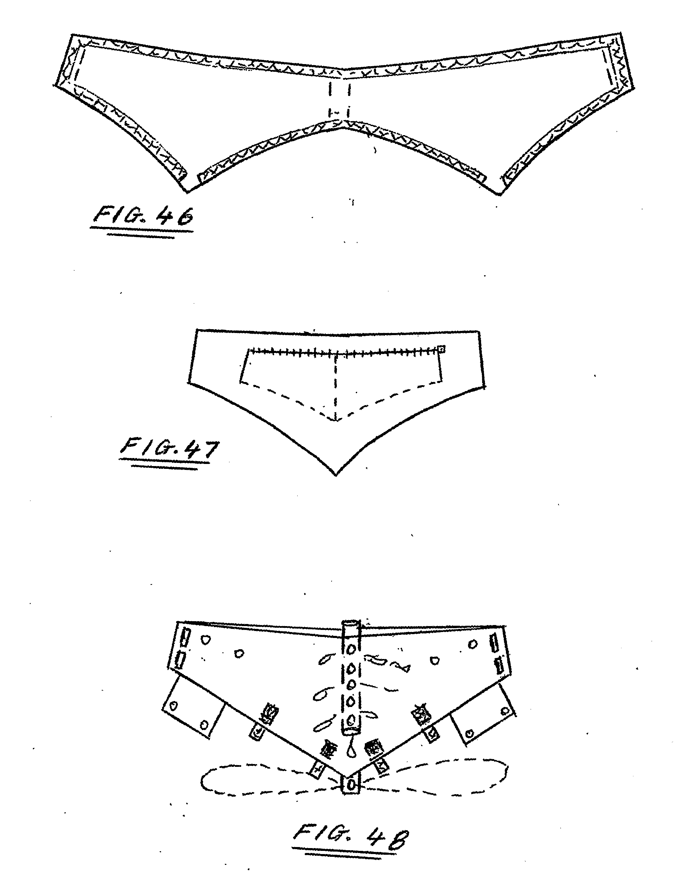

PAGE 10/11, Fig. 46 shows the development of the quilted reusable jacket. Fig. 47 shows the position of the 2 zippable compartments. Fig. 48 is a disposable groin bag with a perforated inlet to aid dispersion.

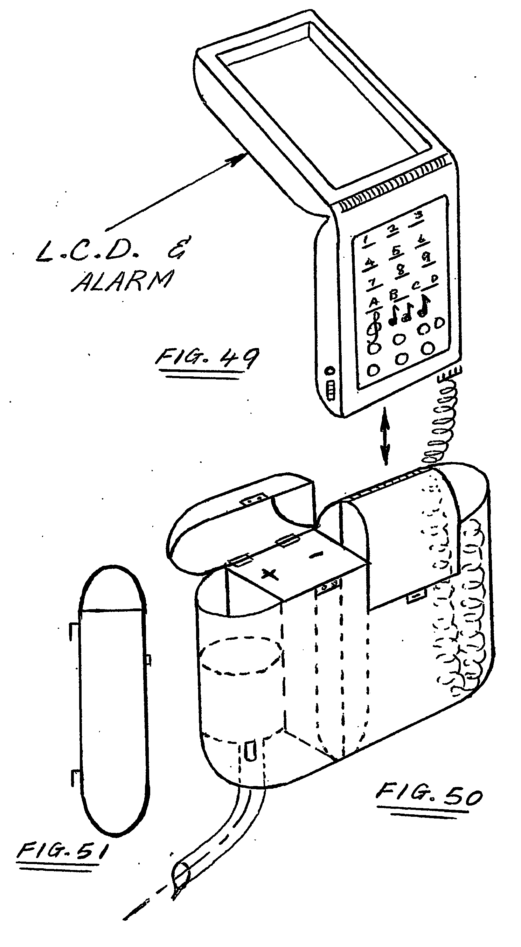

PAGE 11/11, Fig. 49 and Fig. 50 shows an isometric view of the clippable electronic system to be fitted inside the groin bag compartment, revealing the location of the motor, the flexible drive shaft, the battery, the visual L. C. D. unit and control panel enclosed and removed. Fig. 51 is the end view of the control unit.

## Claims

1. An incontinence device for receiving urine from a user, the device comprising:
a urine collection bag adapted to fit the groin region of a user, wherein the urine collection bag has a first compartment for holding urine and a second compartment; a motor;
a urinal having an impeller unit;
a tube connecting the urinal to the first compartment, and
a drive shaft connecting the motor and the impeller unit, **characterized in that**: the second compartment holds the motor; the drive shaft connecting the motor and the impeller unit is flexible; and the flexible drive shaft is separate from the tube.

2. The device according to claim 1, wherein the tube is detachable from the second compartment.

3. The device according to claim 1 or claim 2, wherein the urine collection bag further comprises a flexible, detachable self-closing outlet.

4. The device according to any one of the preceding claims, wherein the urinal is selected from a sheath appendage and an elliptical bowl.

5. The device according to any one of the preceding claims, wherein the urinal further comprises a non-return valve for retaining urine in the urinal.

6. The device according to any one of the preceding claims, wherein the urinal further comprises a liquid detector for controlling the motor.

7. The device according to any one of the preceding claims, wherein the urinal has straps for fastening the urinal to a belt.

## Patentansprüche

1. Inkontinenzvorrichtung zur Aufnahme von Urin von einem Benutzer, wobei die Vorrichtung Folgendes umfasst:
einen Urinsammelbeutel, der an den Leistenbereich eines Benutzers angepasst ist, wobei der Urinsammelbeutel ein erstes Behälterfach zur Aufnahme des Urins und ein zweites Behälterfach umfasst;
einen Motor;
ein Urinal mit einer Pumpradeinheit;
ein Röhrchen, welches das Urinal mit dem ersten Behälterfach verbindet;
eine Antriebswelle, die den Motor und die Pumpradeinheit miteinander verbindet, **dadurch gekennzeichnet, dass** das zweite Behälterfach den Motor aufnimmt, wobei die Antriebswelle, die den Motor und die Pumpradeinheit miteinander verbindet, flexibel ist und die flexible Antriebswelle von dem Röhrchen getrennt ist.

2. Vorrichtung nach Anspruch 1, worin das Röhrchen von dem zweiten Behälterfach abnehmbar ist.

3. Vorrichtung nach Anspruch 1 oder 2, worin der Urinsammelbeutel ferner einen flexiblen, abnehmbaren selbstschließenden Auslass umfasst.

4. Vorrichtung nach einem der vorangegangenen Ansprüche, worin das Urinal aus einem überstülpbaren Fortsatz und einer ellipsenförmigen Schale ausgewählt ist.

5. Vorrichtung nach einem der vorangegangenen Ansprüche, worin das Urinal ferner ein Rückflusssperrventil umfasst, um den Urin in dem Urinal zu halten.

6. Vorrichtung nach einem der vorangegangenen Ansprüche, worin das Urinal ferner einen Flüssigkeitsdetektor zur Steuerung des Motors umfasst.

7. Vorrichtung nach einem der vorangegangenen Ansprüche, worin das Urinal Bänder aufweist, um es an einem Gürtel zu befestigen.

## Revendications

1. Dispositif d'incontinence pour recevoir de l'urine d'un utilisateur, le dispositif comprenant :
un sachet de recueillement d'urine conçu pour s'adapter à la région de l'aine d'un utilisateur, où le sachet de recueillement d'urine présente un premier compartiment pour retenir l'urine et un second compartiment ;
un moteur ;
un urinal ayant une unité d'impulsion ;
un tube reliant l'urinal au premier compartiment, et
un arbre d'entraînement reliant le moteur et l'unité d'impulsion,
**caractérisé en ce que**
le deuxième compartiment retient le moteur ;
l'arbre d'entraînement reliant le moteur et l'unité d'impulsion est flexible ; et
l'arbre d'entraînement flexible est séparé du tube.

2. Dispositif selon la revendication 1, où le tube est détachable du deuxième compartiment.

3. Dispositif selon la revendication 1 ou la revendication 2, où le sachet de recueillement d'urine comprend en outre une sortie flexible, détachable, à auto-fermeture.

4. Dispositif selon l'une des revendications précédentes, où l'urinal est sélectionné à partir d'un appendice de gaine et d'une cuvette eliptique.

5. Dispositif selon l'une des revendications précédentes, où l'urinal comprend en outre une vanne de non-retour pour retenir l'urine dans l'urinal.

6. Dispositif selon l'une des revendications précédentes, où l'urinal comprend en outre un détecteur de liquide pour commander le moteur.

7. Dispositif selon l'une des revendications précédentes, où l'urinal possède des sangles pour fixer l'urinal à une ceinture.
